# EUROPEAN PATENT SPECIFICATION

(11) **EP 2 806 940 B1**
(45) Date of publication and mention of the grant of the patent: **22.05.2019**
(21) Application number: 13740749.0
(22) Date of filing: 24.01.2013
(51) Int. Cl.: A61M 37/00, A61M 5/00, A61N 2/00

(54) **DELIVERY DEVICE**
ABGABEGERÄT
DISPOSITIF D'ADMINISTRATION

(30) Priority: 24.01.2012 AU 2012900262; 18.06.2012 AU 2012902555
(43) Date of publication of application: 03.12.2014
(73) Proprietor: International Scientific PTY Ltd, Leederville, WA 6007 (AU)
(72) Inventor: EDWARDS, Jeffrey D., Nedlands, Western Australia 6009 (AU)
(74) Representative: AWA Sweden AB
(86) International application number: PCT/AU2013/000049
(87) International publication number: WO 2013/110124

(56) References cited:
- WO-A1-2011/156869
- WO-A2-2007/136844
- WO-A2-2008/067290
- WO-A2-2009/102944
- US-A1- 2007 225 676
- US-A1- 2012 046 644
- US-B1- 6 611 707

## Description

### TECHNICAL FIELD

The present invention relates to a device for delivering a measured or predetermined dose of an active agent to a dermal or mucosal surface as defined in claim 1.

### BACKGROUND ART

The following discussion of the background art is intended to facilitate an understanding of the present invention only. The discussion is not an acknowledgement or admission that any of the material referred to is or was part of the common general knowledge as at the priority date of the application.

Micro-needles and other transdermal drug delivery techniques often rely on altering the stratum corneum barrier function prior to the application of a drug or ingredient source. Such two-step processes often involve the poration or disturbance of the stratum corneum barrier as a first step, followed by the application of an active agent over the disturbed area.

However, such two-step process may have negative side effects due to:
a) the need for highly visible and unsightly marking out of the disturbed area, making this approach unsuited to many lifestyle or consumer applications;
b) the necessity for precise alignment, which may require administration by a trained person or doctor;
c) potential unpredictable dose administration due to misalignment;
d) potential for entry of contaminates and disease through the compromised barrier region during exposure of the disturbed region to the environment between steps.

Furthermore, exposure of the disturbed region to the environment could instigate immune system actions that act against the efficient delivery of the active agent.

It is against this background that the present invention has been developed. Document US 6 611 707 B1 discloses a device for delivery of a dose of an active agent to a dermal surface according to the preamble of claim 1.

The present invention seeks to overcome, or at least ameliorate, one or more of the deficiencies of the prior art mentioned above, or to provide the consumer with a useful or commercial choice.

### DESCRIPTION OF EMBODIMENTS

In accordance with a first aspect of the present invention, there is provided a device for delivery of a measured or predetermined dose of an active agent to a dermal or mucosal surface, as defined in claim 1.

The device may further comprise a removable release liner.

The porated disc of the device of the invention has magnetophoretic properties.

The device of the invention may further comprise micro-needles on the porated disc.

The aspects of the invention that provide devices comprising a deformable chamber comprising at least one dome-shaped member defining a reservoir for an active agent and a perforated disc that has magnetophoretic properties and microneedles may further comprise a removable release liner.

The porated disc forms a substantially flat surface to the device which is in contact with the dermal or mucosal surface when the device is in use.

The device of the invention may further comprise an adhesive ring that removably adheres the device to the skin of the subject (wherein the term 'dermal or mucosal surface' can be considered to be interchangeable with the term 'skin' for the purpose of this invention). The adhesive ring is preferably in the form of a flange around the edge of the device adjacent the porated disc, peripheral to the deformable chamber comprising at least one dome-shaped member. The adhesive ring may be an adhesive surface or one of more adhesive regions. Thus, the adhesive ring may be a strip of adhesive material which sticks to the outer rim of the device and, when in use, the dermis or mucosal surface of the user. Alternatively, the adhesive ring may be formed of regions that are adhesive interspersed with regions that are non-adhesive. The adhesive of the ring is preferably compatible with application to skin (i.e. will not cause adverse reactions) and is easily removable from the skin after use.

When using the device, the adhesive ring allows the device to be held in the correct location on the skin during deformation of the deformable chamber and delivery of the active agent. The adhesive ring may hold the device to the skin for a brief period during rapid delivery of the active agent, or may hold the device against the skin for longer periods for slower or more sustained release of active agents.

Alternatively, the device of the invention may be adhered to the skin surface by the viscose properties of the active agent delivered by the device. For example, if the active agent is suitably sticky or viscous, the interaction of the active agent with the skin will ensure that the delivery device does not move substantially from the site of application during use.

The device of the invention may further comprise a transparent window ring around the periphery of the deformable chamber, more preferably within the region defined by the adhesive ring if the device has one. This transparent window ring permits visual identification of delivery of the active agent, as it fills with active agent when the deformable chamber is deformed by finger pressure and the active agent moves from the deformable chamber to the region between the porated disc and the skin. Preferably, the amount of active agent in the transparent window ring is an indication of the amount of active agent which has moved from the deformable chamber. Thus, if only some of the active agent has moved from the deformable chamber to the region between the porated disc and the skin, the transparent window ring will only be partially full of active agent. Once the transparent window ring is full of active agent, this will aid in indicating to the user that substantially all of the active agent has moved from the deformable chamber to the region between the porated disc and the skin.

The transparent window ring may be fully transparent around its diameter, or may be semi-transparent. The transparent window ring may alternatively have sections that are transparent, and sections that are not transparent or are opaque. Generally, any configuration is allowable, such that the user can visualise the movement of the active agent into the transparent window ring to determine that the active agent has moved from the deformable chamber to the region between the porated disc and the skin and then into the transparent window ring.

The device may additionally comprise an absorbent delivery surface, located between the porated disc and the skin when the device is in use. If an absorbent delivery surface is present, then in use the deformation of the deformable chamber moves the active agent from the deformable chamber, through the pore(s) in the porated disc and into the absorbent delivery surface. The active agent would pass through the absorbent delivery surface through holes or pores in the absorbent delivery surface, by wicking due to capillary action, or simply by forcing the agent through the absorbent delivery surface due to the pressure of the deformation of the deformable chamber. The active agent is then delivered to the skin through the absorbent delivery surface. The absorbent delivery surface may comprise a sponge, foam, pad or fabric material.

The absorbent delivery surface may be covered with a removable liner before use, in which case the removable liner is removed before the device is placed on the skin. Alternatively, the absorbent delivery surface may actually comprise the removable liner, in which case the removable liner is not removed before use, but simply forms a delivery surface when the deformable chamber is deformed and the active agent released into the removable liner/absorbent delivery surface.

Preferably, the device of the invention, if provided with an absorbent delivery surface, is moved over the skin to deliver the active agent over an area of skin surface larger than the area of the device itself, i.e. the device is rubbed over a small or large area of skin to apply the agent to an area. The movement is preferably in an arc-like, semi-circular, circular or orbital manner.

The device of the invention may also be moved over the skin to deliver the active agent over an area of skin surface larger than the area of the device itself if the device does not have an absorbent delivery surface, that is if the surface of the porated disc or skin facing member in contact with the skin or mucosal surface is substantially non-absorbent (eg if it was made from smooth plastic). In such cases, the movement of the device would still apply the active gent over an area of skin surface larger than the area of the device itself.

According to the invention, the porated disc of the device has magnetophoretic properties, and the combination of the magnetophoretic properties of the porated disc and the reciprocal, rotational or orbital movement of the device over the skin imparts a movement to the magnetic elements of the device such that the dermal or mucosal surface in contact with the active agents will be subject to alternating polarities of magnetic field and alternating magnetic gradients in response to said reciprocal, rotational or orbital movement.

The deformable chamber is preferably substantially circular or ovoid in horizontal section in relation to the longitudinal plane of the porated disc when in use against the skin, with the dome-shaped member that is to be deformed being convex. However, the deformable chamber can be square, hexagonal or rectangular, or any other shape in horizontal section. The dome-shaped member that is to be deformed may be smoothly domed into a convex form, or may comprise an outer face surrounded by a tapering face, wherein the outer face is the surface that is to be deformed. The chamber is preferably between about 15mm and 50mm in diameter and the height or diameter of the deformable chamber is preferably between about 12mm and 45mm in vertical section in relation to the longitudinal plane of the porated disc when in use against the skin.

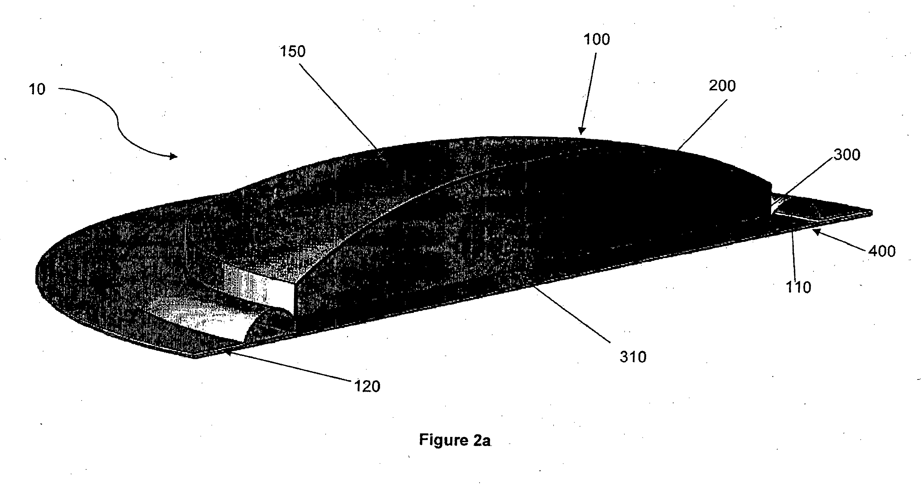

The deformable chamber may have one dome-shaped member and be of a semi-circular shape in vertical section in relation to the longitudinal plane of the porated disc when in use against the skin, with the porated disc forming the substantially flat surface of the semi-circle and the dome-shaped member that is to be deformed forming the curve of the semi-circle (see, for example, Figure 2a).

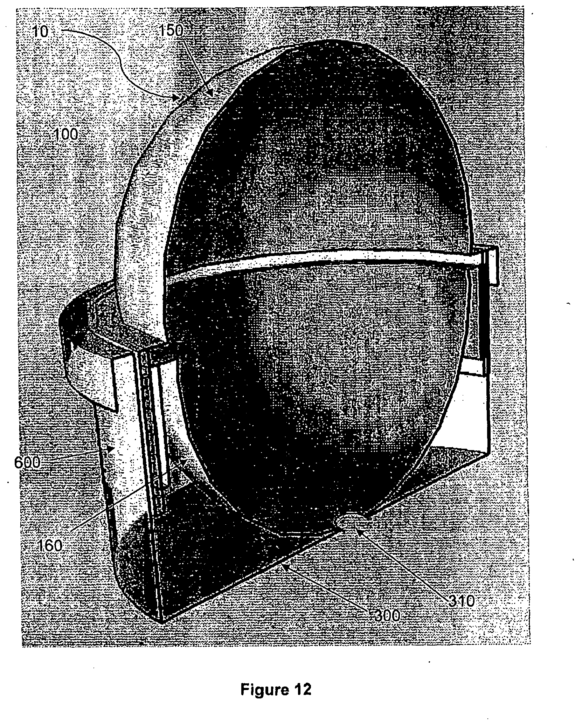

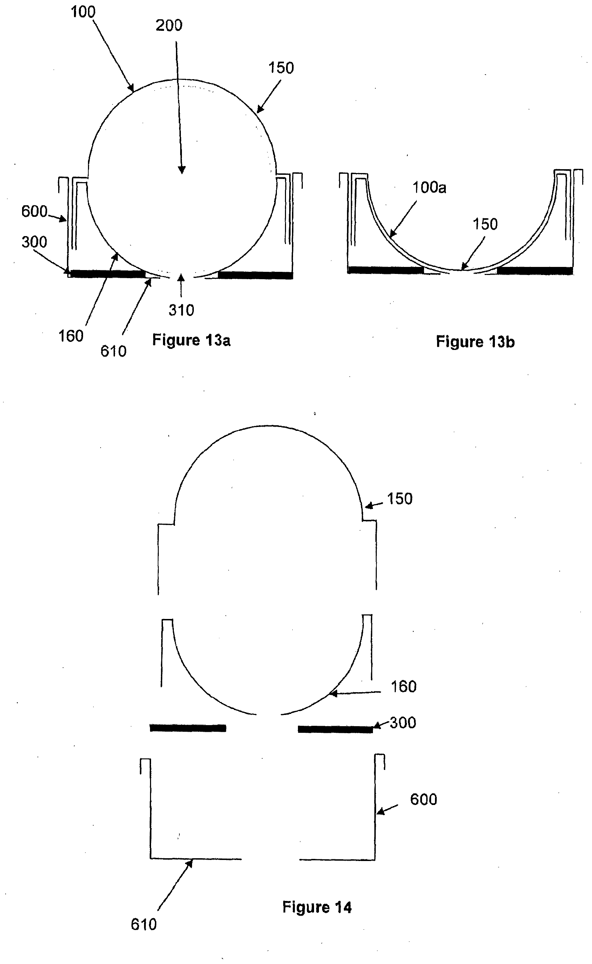

Alternatively, the deformable chamber may comprise two dome-shaped members and be substantially spherical in vertical section in relation to the longitudinal plane of the porated disc when in use against the skin. The deformable chamber may be made from two dome-shaped members, with at least the dome-shaped member distal to the skin surface when in use being deformable (see for example, Figure 12 and 13a) or the deformable chamber may be made from a single sphere. If the deformable chamber is spherical in vertical section, the porated disc will be located between the chamber and the skin surface when in use.

Preferably, the device further comprises a wall section that rises from the outer edge of the porated disc vertically in relation to the longitudinal plane of the porated disc when in use against the skin and which extends at least as far as the widest longitudinal diameter of the deformable chamber. The wall section preferably contacts the deformable chamber with sufficient force to hold the deformable chamber within the interior of the wall section (see, for example, Figure 12 and 13a). Alternatively, the wall section may be adhered to the deformable chamber by an adhesive, heat, electrostatic forces, Van der Waals forces (eg dry adhesion, dispersive forces) etc.

The wall section may further incorporate a skin facing member that extends laterally from the edge of the wall section and is substantially parallel with the porated disc and which extends between the disc and the skin of the subject when the device is in use (see, for example, Figure 13a). The skin facing member should have pores that correspond with the pores of the porated disc, to allow the active agent to pass through the skin facing member to the skin. Alternatively, the porated disc may be adhered to or integral with the wall section on the edge of the wall section that is adjacent to the skin when in use and thus the porated disc forms the skin facing member (see, for example, Figure 14). The wall section may be adhered to the porated disc by an adhesive, heat, electrostatic forces, Van der Waals forces (eg dry adhesion, dispersive forces) etc.

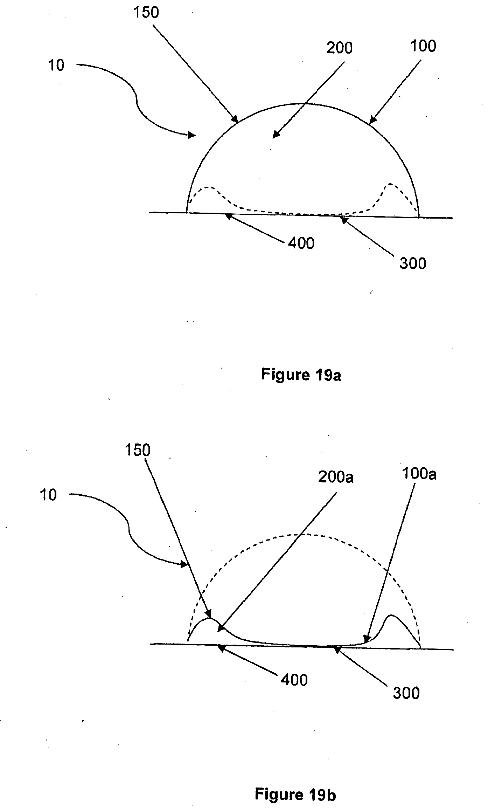

Optionally, the dome-shaped member of the deformable chamber is in a tensioned state when manufactured, such that external pressure on the dome-shaped member causes the dome-shaped member to deform by rebounding into a relaxed state (see, for example, Figure 19a which shows the dome-shaped member of the deformable chamber in a tensioned state and Figure 19b which shows the dome-shaped member having deformed into a relaxed state). Generally, the external pressure is provided by a subject's finger on the point of the dome-shaped member that is furthest from the skin. The dome-shaped member of the deformable chamber that is in a tensioned state is preferably the dome-shaped member distal to the skin surface when in use if the device has two dome-shaped members.

According to the invention, the volume of the deformable chamber is predicably changed by the application of finger pressure to the deformable dome-shaped member, i.e. it is dimensionally unstable and will change shape but will not rupture or break when pressure is applied. By "predictably changed", it is anticipated that the shape of the chamber is altered in a predictable way by the application of finger pressure, so that the crown or top of the dome-shaped member collapses downwards to reduce the volume of the deformable chamber. Generally, the volume of the deformable chamber is preferably between about 0.1ml and 5 ml. If the chamber is semi-circular in shape in vertical section in relation to the longitudinal plane of the porated disc when in use against the skin, then preferably the volume is between 0.1 and 2 ml. If the chamber is spherical in shape in vertical section in relation to the longitudinal plane of the porated disc when in use against the skin, then the volume is preferably between about 2 and 5 ml.

The nature of the deformable chamber is such that, when the deformable chamber is deformed, substantially all of the measured volume of the active agent is dispensed and dispelled from the chamber due to the dome-shaped member of the chamber distal from the skin when in use deforming to adopt the shape of either (a) the substantially flat porated disc if the chamber is semi-circular in shape in vertical section in relation to the longitudinal plane of the porated disc when in use against the skin or (b) the shape of the second dome-shaped member adjacent to the skin if the chamber is spherical in shape in vertical section in relation to the longitudinal plane of the porated disc when in use against the skin. The porated disc of the delivery device may comprise a magnetic array comprising one or more pairs of displaced dipolar magnetic elements. Alternatively, the porated disc can be a single magnet.

The porated disc preferably has the same shape and diameter as the deformable chamber. For example, if the deformable chamber is semi-circular in shape in vertical section in relation to the longitudinal plane of the porated disc when in use against the skin, then the deformable chamber preferably occupies the entire open region of the deformable chamber that is proximal to the skin. The porated disc is preferably substantially circular or ovoid in shape, the same as the deformable chamber. However, the porated disc can be square, hexagonal or rectangular, or any other shape.

As the porated disc has magnetophoretic properties, the magnetic elements may be constructed using a range of magnetic materials exhibiting ferromagnetic properties. Such materials may include an iron compound (e.g. a ferrite such as barium ferrite, magnetite, or mild steel), cobalt compound or nickel compound, optionally with a metalloid component such as boron, carbon, silicon, phosphorus or aluminium. Alternately, rare-earth materials such as neodymium or samarium-cobalt may also be used. Such ferromagnetic materials may be deployed as rigid elements within the device or encapsulated in a flexible matrix such as rubber or silicone.

Preferably, the magnetic elements are formed of various arrangements of displaced dipolar magnetic pairs, allowing diamagnetic repulsion to be maintained in the presence of dielectric polarization, thus providing an effective means of influencing molecular movement and permeation enhancements of barriers during the delivery of active agents. This can be achieved by:
a) juxtaposing two or more magnetic fields generated by one or more pairs of displaced dipolar magnetic elements to achieve a spatially varying gradient of magnetic flux;
b) moving two or more magnetic fields generated by one or more pairs of displaced dipolar magnetic elements over a fixed point to achieve a temporally varying gradient of magnetic flux, or
c) a combination of (a) and (b).

Each pair of displaced dipolar magnetic elements in the porated disc of the present invention may, for the purposes of visualisation, be thought of in terms of a single traditional rod dipolar magnetic that is cleaved or broken around its central point and the two resulting sections rotated through 180⁰ and brought together such that the opposite poles are adjacent. The result is a dipolar pair, displaced horizontally so as to provide both a perpendicular and a horizontal magnetic flux gradient.

In each pair of displaced dipolar magnetic elements, each individual dipole provides diamagnetic repulsion to the active agents that are to be transported across the biological barrier, whilst the horizontal flux between each dipole pair acts to polarize the dielectric properties of the target tissue such as the dermal or mucosal surface, inducing permeation changes.

As the porated disc of the present invention is magnetophoretic, the magnetic porated disc may comprise one pair of displaced dipolar magnetic elements, or may preferably comprise a number of pairs of displaced dipolar magnetic elements. The terms "pair" and "pairs" are used variously in the present specification, and reference to a single pair of displaced dipolar magnetic elements may be taken to also refer to multiple pairs of displaced dipolar magnetic elements. The polarity of one pair of displaced dipolar magnetic elements may be in the same orientation as that of the neighbouring pair of displaced dipolar magnetic elements (i.e. the series may comprise [NS][NS][NS]) or may be in the opposite orientation (i.e. the series may comprise [NS][SN][NS]). T

The device of the present invention may be moved over the mucosal or dermal surface of the user, through manual operation (ie through normal consumer actions of rubbing). The movement is preferably in an arc-like, semi-circular, circular or orbital manner. The frequency of movement may be in the order of 1 Hz to 5 Hz. In such cases, if the porated disc is magnetic, the strength of the magnet field produced by each element of the magnet array should be between about 100 and 500 Gauss.

Generally, each pair of displaced dipolar magnetic elements of the present invention has a horizontal offset between centres of between 1 and 5 millimetres. As a result, pairs of displaced dipolar magnetic elements may be disposed at a repetition rate of between 2 and 10 dipolar pairs per centimetre.

Preferably, the poles in a particular spatial region are between 1.0 mm to 10mm apart, more preferably, the poles are between 2.0 mm to 5.0mm apart.

In another aspect of the invention, the magnetic flux of each magnetic pole is between about 10 Gauss and about 1000 Gauss. Preferably, the flux of each pole is between about 100 Gauss to about 600 Gauss, most preferably about 125 to 450 Gauss.

In another aspect, the difference or delta flux between the magnetic flux of two adjacent poles of opposite polarity is between about 200 Gauss and about 2000 Gauss. More preferably, the difference between the magnetic flux of two adjacent poles of opposite polarity is between about 600 Gauss to about 1400 Gauss, most preferably about 500 to 1800 Gauss.

When the magnetophoretic porated disc comprises at least two sets of pairs of displaced dipolar magnetic elements wherein the alignment of the first set of displaced dipolar magnetic elements is angularly offset relative to the alignment of the second set of displaced dipolar magnetic elements, the orientation of the first set of dipolar pairs is preferably between about 1° and 90° relative to the second set of dipole pairs. Preferably, the degree of angular offset is at least 10°, more preferably at least 45°, most preferably between about 45° and 90°.

Such magnetophoretic porated discs with at least two sets of pairs of displaced magnetic elements may have a different number of dipolar pairs disposed in the first set from that in the second set. For example, the first set of dipolar pairs may have two dipolar pairs per centimetre whilst the second set may have five dipolar pairs per centimetre.

Where a different number of dipolar pairs is used in each set of dipolar pairs in differing orientations, the magnetic fields will be complex and exhibit different flux densities in each orientation, as the fields produced by the first set of dipolar pairs will sum with that of the second set of dipolar pairs at the points of constructive and destructive interference and by doing so provide a net field of higher magnetic strength, higher magnet flux and higher magnetic gradient, all of which will add to the utility of the present invention.

The purpose of multiple intersection orientations is twofold:
a) to accommodate non-linear movements either by users or by devices. Then induction effect is reliant on the target barrier dermal or mucosal surface being influenced by an alternating field, which only happens when the device is tracked across the barrier at 90° to the alignment of the elements. To accommodate a circular motion, the arrays are aligned so to produce an AC like induction, irrespective of the direction of motion; and
b) to induce opposing charges in adjacent areas of the dermal or mucosal surface so as to produce streaming potentials to accommodate pathways that are not perpendicular to the field flux, such as vertical shunts or pathways.

The magnetophoretic porated disc of the device may further comprise more than two sets of dipolar pairs. The orientation of these further sets of pairs of displaced dipolar magnetic elements may align with the first set of pairs of displaced dipolar magnetic elements and thus be angularly offset to the second set of pairs of displaced dipolar magnetic elements, or may align with the second set and thus be angularly offset to the first set of pairs of displaced dipolar magnetic elements. Further orientations and arrangements of sets of pairs of displaced dipolar magnetic elements may be provided which align with either the first or second sets of displaced dipolar magnetic elements. For example, a many layered magnetophoretic porated disc may be provided which comprises a number of orientation of pairs of displaced dipolar magnetic elements stacked on top of each other, each one aligned in a different orientation to the array below (e.g. each set aligned perpendicular to the set below).

The porations in the porated disc may comprise one single pore, or a number of pores to allow the active agent to pass through when the deformable chamber is deformed. The size or diameter of the pore(s) may vary, as a function of the speed with which one wants to deliver the active agents, or the total amount of active agents one wants to deliver from the deformable chamber. Depending on intended use, the diameter of the pore(s) may range from 1.0 to 10.0 mm. The larger openings may require the use of hydrogel in the deformable chamber, so the active agent does not immediately run out of the deformable chamber when the removable release liner is removed. The geometry of the pore(s) may be in the form of many shapes, i.e., round, rectangular, elliptical, square, etc.

If present, the removable release liner is preferably attached to the surface of the porated disc, the skin facing member and/or the edge of the deformable chamber that is proximal to the skin surface when the delivery device is in use, such that it seals the pores in the porated disc and prevents active agent from passing through. The liner may be adhesive or semi-adhesive such that it adheres to the edges of the deformable chamber, some or all of the surface of the porated disc, and/or the edges of the transparent window ring. Alternatively, the removable release liner may be bonded by heat, electrostatic forces, Van der Waals forces (eg dry adhesion, dispersive forces) etc to the previously mentioned edges and/or surfaces. The removable release liner may be further adhered to the adhesive ring that optionally surrounds the optional transparent window ring. In this case, removal of the removable release liner would preferably expose the adhesive on the adhesive ring and allows the adhesive to be applied to the skin of the subject.

Alternatively, the pores in the porated disc may comprise thinner regions than the majority of the porated disc, such that the pressure of the active agent on these regions during deformation of the deformable chamber causes the thinner regions to rupture or tear and allow the active agent to pass through the newly created pores in the porated disc.

The device of the present invention may be applied to the skin, the deformable chamber deformed and then the device removed immediately after delivery of the active agent. Alternatively, the device may be left applied to the skin for a period after deformation of the chamber and delivery of the active agent. In this second case, it would be advantageous for the device to comprise the optional adhesive ring. However, the adhesive ring can also aid in placement of the device even if it is to be removed immediately after delivery of the agent, and would assist in ensuring that the active agent is delivered to precisely the correct location - especially if microneedles are also used, wherein the adhesive assists in retaining the active agent over the location of the poration created by the microneedles.

The microneedles of the present invention, if present, are preferably of the solid type, with a length between about 150µm and 1000µm and a base dimension of 50µm to 150µm. A range of commercially available microneedles of various lengths and widths may be used. Many shapes and sizes of microneedles have been explored and found to provide benefit, as presented in "Microneedle technology for advanced drug delivery: Evolving vistas" (CRIPS Vol. 9 No. 1 January-March 2008). In another example, the microneedles may be made of silk (see, for example "Fabrication of Silk Microneedles for Controlled-Release Drug Delivery" Advanced Functional Materials [2011]). Most preferably, the needles are formed from surgical steel and are about 700µm long and 260µm wide, with a tip that is 250µm long and at a 55° angle.

The device of the present invention may be pre-loaded with a measured dose of an active agent, on alternatively a measured dose of the active agent may be delivered into the reservoir of the deformable chamber immediately before use of the delivery device.

The deformable chamber should be made of a substance that can be deformed by finger pressure without rupturing or breaking. Suitable materials for the chamber can include but are not limited to: ceramics; metals such as titanium, aluminum or steel; plastics such as polyolefins, barex, styrene, polyesters, polyacrylics, vinylpolymers, polyamides, polyfluorocarbons, polyimides, polylactams, polyaramides, polycarbonates, polysulfones, polyethylene, polypropylene, nylon, polyvinyl chloride, polyvinylidiene chloride or combinations or composites thereof.

Preferably, the device of the present invention is designed for single use applications, i.e. the device is loaded with one dose of active agent (either during manufacture or just prior to use), the dose is applied to the skin of the user and the device is disposed of. The device may also be manufactured at one location, minus the active agent. The device may then be delivered to another location or manufacturer and loaded with active agent prior to purchase and use by a user. Again, it is envisaged that the device is disposed of after this use, as the deformable chamber has been deformed. However, the device may be re-used by restoring the deformable chamber to its original form and reloading the reservoir of the deformable chamber with active agent.

Active agents that may be delivered by the device of the present invention include vaccines and therapeutic compounds for the treatment or prevention of a range of conditions. The active agents may be pre-loaded into the device (i.e. the deformable chamber may be filled with the active agent during manufacture of the device, or after manufacture but before delivery to the user). Alternatively, the user of the device may fill the deformable chamber of the device with the active agent immediately before use.

Suitable active agent(s) that can be delivered by the invention include any active agent(s) exhibiting negative magnetic susceptibility and any active agent(s) having therapeutic, cosmetic, restorative, antimicrobial, anti-fungal, cleaning or disinfectant beneficial properties when administered to a dermal or mucosal surface as described herein. When the invention is employed to assist in the passage of active agents across a biological barrier such as such as skin the class of active agents, include, for example, proteins, peptides, nucleotides, anti-obesity drugs, corticosteroids, analgesics, anti-fungal agents, oncology therapies, cardiovascular agents, anti-inflammatory agents, non-steroidal anti-inflammatory agents, anti-arrhythmic agents, antibiotics, anticoagulants, antidepressants, antidiabetic agents, antiepileptics, antihistamines, anti-hypertension agents, anti-neoplastic agents, immunosuppressants, anti-thyroid agents, antiviral agents, sedatives, astringents, beta-adrenoceptor blocking agents, diuretics, muscle reactants, prostaglandins, sex hormones, anti-allergic agents, stimulants, vasodilators, xanthenes, antioxidants, vitamins, nutrients, skin restorative agents, hair care or restorative agents and those active agents delivered as nutraceuticals, cosmeceutical or cosmetics to or through a biological barrier such as skin.

Additionally, the device of the present invention may be used to deliver agents such as nicotine and nicotine replacement agents such as nicotinic agonist, acetyl choline, varenicline tartrate, bupropion etc.

The device may also be used for the delivery of vaccines and immunogenic agents.

More specific groups of active agents that may be delivered by the device of the present invention include: colouring agents including coloured cosmetics, concealing agents, skin whitening agents, tanning agents; cleaning agents including exfoliants, dermabrasives, anti-bacterials, anti-fungals, sloughing agents, pore treatment agents, oils, creams, gels and serums; moisturising agents, rehydrating agents and skin nourish agents; skin treatment agents for inflammation, redness, pimples and bacterial infections; antiperspirants, deodorants, and perfumes; fillers, plumping agents and agents that alter the skin's topography; anti-inflammatory agents, analgesic agents and anaesthetic agents

Non-limiting examples of pharmaceutical and biopharmaceutical active agents which could be delivered to the skin using the device of the present invention include:
a) steroids such as sulfonamides, triamcinolone, betamethasone, budesonide, cortisone, dexamethasone, hydrocortisone, methylprednisolone, prednisolone, prednisone sodium phosphate, fluorometholone, rimexolone, medrysone alcohol, 11 -desoxcortisol, and anecortave acetate and the like
b) anti-inflammatory agents such as hormonal agents, clobetasol, dexamethasone, acetyl salicylic acid, glycyrrhizic acid or glycyrrhetic acid, hydrocortisone, prednisolone, prednisone, non-hormonal agents, allopurinol, aspirin, indomethacin, phenylbutazone and the like;
c) antibiotics such as the cephalosporins, chloranphenical, gentamicin, Kanamycin A, Kanamycin B, the penicillins, ampicillin, streptomycin A, antimycin A, chloropamtheniol, metromidazole, oxytetracycline penicillin G, the tetacyclines and the like;
d) anti-acne agents, such as salicylic acid and benzoyl peroxide;
e) antimicrobial or antifungal agents such as, for example, caprylyl glycol, triclosan, phenoxyethanol, erythromycin, tolnaftate, nystatin or clortrimazole;
f) chelating agents, such as EDTA and the like;
g) topical analgesics, such as benzocaine, tetracaine, lidocaine or procaine and the like;
h) peptides with either therapeutic benefit or cosmetic benefit comprising between two and 20 amino acid residues, preferably, between three and 10 amino acid residues (cosmetic peptides such as palmitoyl pentapeptide or argireline which have beneficial effect on skin cells eg whitening, free-radical scavenging, anti-aging, stimulation of collagen synthesis, moisturizing, antimicrobial, anti-inflammatory, or anti-irritant) and the like.
i) cosmetic agents such as Botulinum toxin and derivatives and variants.
j) Muscle relaxants such as Methocarbamol
k) Joint, cartilage and articular surface agents such as hyaluronic acid, chondroitin sulphate, glucosamine, glycosaminoglycans, aggrecans and similar agents.

Non-limiting examples of neutraceutical active agents which could be delivered to the skin using the device of the present invention include:
a) Vitamins and nutrients including essential amino acids and the like;
b) Electrolyte replacements such as potassium chloride and the like;
c) Antioxidants or free-radical scavengers: such as ascorbic acid, its fatty esters and phosphates, tocopherol and its derivatives, N-acetyl cysteine, sorbic acid and lipoic acid and the like;

Non-limiting examples of cosmetic active agents which could be delivered to the skin using the device of the present invention include:
a) Moisturising agents and emollients: occlusive agents e.g. hydrocarbons such as petrolatum, silicone containing agents such as dimethicone, cyclomethicone, fatty acids and alcohols such as lanolin acid or alcohol, sterols such as cholesterol, waxes and fats such as cocoa butter, carnuba wax and bees wax; humectants e.g. glycosamines such as hyaluronic acid, glycerine, honey, urea, lactic acid, α-hydroxy acids, propylene glycol etc;
b) Anti-aging compounds: such as retinoids or hydroxy acids;
c) Deodorants: including alcohol based deodorants; deodorants containing antimicrobial agents such as triclosan or metal chelating agents; perfumes and essential oils;
d) Antiperspirants: including aluminium based compounds such as aluminium chloride, aluminium chlorohydrate, and aluminium-zirconium compounds (aluminium zirconium tetrachlorohydrex gly and aluminium zirconium trichlorohydrex gly); potassium alum and ammonium alum;
e) Fragrances: such as essential oils, musk, alcohols (eg furaneol, menthol), esters (eg fructone, ethyl methylphenylglycidate), ketones (eg dihydrojasmone), lactones (eg coconut odour, jasmine lactone);
f) Astringents: such as clove oil, menthol, camphor, eucalyptus oil, eugenol, menthyl lactate, witch hazel distillate, aluminium based compounds and the like;
g) Skin lightening agents: such as liquorice, ascorbyl phosphates, hydroquinone or kojic acid and the like;
h) Sun protecting agents (organic or inorganic): such as avobenzone, oxybenzone, octylmethoxycinnamate, titanium dioxide or zinc oxide;
i) Exfoliating agents (chemical or physical): such as N-acetyl glucosamine, mannose phosphate, hydroxy acids, lactobionic acid, peach kernels, or sea salts and the like;
j) Self-tanning agents: such as dihydroxyacetone;
k) Colouring agents for skin and mucosal surfaces;
l) Plumping agents and fillers: such as hyaluronic acid or hyaluronate.
m) Other agents such as aloe vera.

Additional agents that could be delivered to the skin surfaces include additional nutritional type ingredients, such as vitamins, minerals, amino acids, vitamin E, and folic acid; sensate ingredients, such as those providing cooling (such as menthol), tingle, or heat sensations (such as capsaicin or capsicum oil); colorants or other aesthetic agents; and combinations thereof. Essential oils may also be delivered by the present invention, such as oils of lavender, rose, rosemary, spearmint, peppermint, wintergreen, eucalyptus, lemon, lime, grapefruit, and orange.

The above list of active agent(s) may be applied in a controlled manner, using the device of the present invention. This list is not exhaustive. Preferably, any active agent(s) that can be delivered systemically or topically can potentially be delivered using the present invention.

The active agent may be in the form of a solid, gel, paste, liquid, thermo-reversible gel or paste, etc.

While the active agent(s) may be provided and used alone with the device, in many situations the active agent will be included in a formulation either alone or in combination with one or more other active agents. Where the formulation is to provide a pharmaceutical and/or biopharmaceutical benefit, the number of active agent(s) included in the formulation may preferentially be quite selective. Where the formulation provides a nutraceuticals, cosmetic and/or cosmeceutical effect, the number of active agents may be much greater in number.

The formulation employed in the delivery process may include additives such as other buffers, diluents, carriers, adjuvants or excipients. Any pharmacologically acceptable buffer that is magnetically inert or neutral or which has a magnetic susceptibility that is either paramagnetic in nature or greater than that of the active agent(s) being delivered, may be used, e.g., tris or phosphate buffers. Other agents may be employed in the formulation for a variety of purposes. For example, buffering agents, preservatives, co-solvents, surfactants, oils, humectants, emollients, chelating agents, stabilizers or antioxidants may be employed. Water soluble preservatives which may be employed include, but are not limited to, benzalkonium chloride, chlorobutanol, thimerosal, sodium bisulfate, phenylmercuric acetate, phenylmercuric nitrate, ethyl alcohol, methylparaben, polyvinyl alcohol, benzyl alcohol and phenylethyl alcohol. A surfactant may be Tween 80. Other vehicles that may be used include, but are not limited to, polyvinyl alcohol, povidone, hydroxypropyl methyl cellulose, poloxamers, carboxymethyl cellulose, hydroxyethyl cellulose, purified water, etc. Tonicity adjustors may be included, for example, sodium chloride, potassium chloride, mannitol, glycerin, etc. Antioxidants include, but are not limited to, sodium metabisulfite, sodium thiosulfate, acetylcysteine, butylated hydroxyanisole, butylated hydroxytoluene, etc.

The indications, effective doses, contra-indications, vendors etc, of the active agents in the formulations are available or are known to one skilled in the art.

The active agents may be present in individual amounts of from about 0.001 to about 5% by weight and preferably about 0.01% to about 2% by weight. However, it is contemplated that the active agents may be present in individual amounts greater than this, for example up to 100%.

Suitable water soluble buffering agents that may be employed include sodium carbonate, sodium borate, sodium phosphate, sodium acetate, sodium bicarbonate, etc., as approved by the US FDA for the desired route of administration. These agents may be present in amounts sufficient to maintain a pH of the system of between about 2 to about 9, preferably about 4 to about 8, more preferably 4.5, 5, 5.5, 6, 6.5, 7 or 7.5 (or any pH in between). As such the buffering agent may be as much as about 5% on a weight to weight basis of the total formulation. Electrolytes such as, but not limited to, sodium chloride and potassium chloride may also be included in the formulation where appropriate.

The active agents to be delivered using the device of the present invention may be provided in a matrix layer. If the active agent delivered by the device of the present invention is contained within a matrix, the matrix preferably allows the active agent to diffuse or exit the matrix in some manner and contact the dermal or mucosal barrier, perhaps by being transmitted through a cover portion of the first portion to the dermal or mucosal barrier.

The matrix is preferentially prepared from a polymer or copolymer prepared from e.g., polyisobutylene, ester of polyvinyl alcohol, polyacrylic and polymethacrylic acid esters, natural rubber, polymers of styrene, isoprene, and styrene-butadiene or silicone polymers, resin components, such as, saturated and unsaturated hydrocarbon resins, derivatives of abietyl alcohol and of beta-pinene, plasticizers, such as phthalic acid esters, triglycerides and fatty acids, as well as a series of other substances known to those skilled in the art.

Matrix biocompatible polymers that might be used in the invention include compounds such as polycaprolactone, polyglycolic acid, polylactic acid, polyanhydrides, polylactide-co-glycolides, polyamino acids, polyethylene oxide, acrylic terminated polyethylene oxide, polyamides, polyethylenes, polyacrylonitriles, polyphosphazenes, poly(ortho esters), sucrose acetate isobutyrate (SAIB), and other polymers such as those disclosed in U.S. Patent Nos. 6,667,371; 6,613,355; 6,596,296; 6,413,536; 5,968,543; 4,079,038; 4,093,709; 4,131,648; 4,138,344; 4,180,646; 4,304,767; 4,946,931.

The matrix containing the active agents may also be prepared from thermosetting polymers such as tetra-substituted ethylene diamine block copolymers of ethylene oxide and propylene oxide (e.g., poloxamine); polycarbophil; and polysaccharides such as gellan, carrageenan (e.g., kappa-carrageenan and iota-carrageenan), chitosan and alginate gums.

The matrix may also be a hydrogel, being a gel prepared with hydrophilic polymers, and these materials are well known in the art, frequently being used as part of biomedical electrodes, such as are described in U.S. Pat. Nos. 6,631,294 and 6,845,272. Examples of hydrophilic polymers useful for the preparation of hydrogels are polyacrylate, polymethacrylate, polyacrylamide, polyvinyl alcohol), poly(ethylene oxide), poly(ethylene imine), carboxy-methylcellulose, methylcellulose, poly(acrylamide sulphonic acid), polyacrylonitrile, poly(vinyl-pyrrolidone), agar, dextran, dextrin, carrageenan, xanthan, and guar. The preferred hydrogels are acrylates and may be, for example, preferably made from acrylic esters of quaternary chlorides and/or sulfates or acrylic amides of quaternary chlorides; polymers of this type are disclosed in U.S. Pat. No. 5,800,685. The hydrophilic polymers will generally constitute from about 1 to about 70%, preferably about 5 to about 60%, more preferably about 10 to about 50%, by weight of the hydrogel.

In a highly preferred form of the invention, a topical formulation for delivery to a subject is prepared by selecting a desired amount of active agent. The agent is then preferably placed in a suitable delivery matrix. The amount of the active agent to be administered and the concentration of the compound in the topical formulation depends upon the diluent, delivery system or device selected, the clinical or cosmetic condition of the subject, the side effects and the stability of the active agent in the matrix.

The minimally invasive active agent delivery devices of the present invention, for the delivery of drugs and vaccines, provide a safe, efficacious, economic and consistent means for administering agents for enabling therapeutic responses.

The present invention further provides a method for delivering a cosmetic agent to a dermal or mucosal surface comprising the steps of: applying a device for delivering an active agent to a dermal or mucosal surface to the dermal or mucosal surface, wherein the device comprises: a deformable chamber comprising at least one dome-shaped member defining a reservoir for an active agent and a porated disc.

The present invention provides a kit comprising the inventive device according to claim 1.

In another aspect, the kit comprises a delivery device for delivering an active agent to a dermal or mucosal surface comprising: (a) a deformable chamber comprising at least one dome-shaped member defining a reservoir for an active agent and a porated disc; and (b) a separate container containing an effective amount of the active agent to be administered.

By an "effective amount" or "effective dosage" of a substance it is intended to mean an amount that will elicit a desired response in a subject, including, but not limited to, an immunostimulatory response in the case of an allergen or vaccine, or other therapeutic or diagnostic response.

In a further aspect, the kit comprises a delivery device for delivering an active agent to a dermal or mucosal surface comprising: (a) a deformable chamber comprising at least one dome-shaped member defining a reservoir for an active agent and a porated disc; and (b) an effective amount of the active agent to be administered, wherein the active agent is pre-loaded into the deformable chamber.

To use a kit as envisioned by certain aspects of the instant invention, the practitioner would break a hermetic seal to provide access to the delivery device and optionally, the active agent (eg vaccine or immunogenic or therapeutic composition). The active agent may be preloaded into the deformable chamber of the delivery device in any suitable form, including but not limited to gel, paste, oil, emulsion, particle, nanoparticle, microparticle, suspension or liquid, in a suitable dosage. The active agent may be separately packaged within the kit package, for example, in a reservoir, vial, tube, blister, pouch, patch or the like. One or more of the constituents of the formulation may be lyophilized, freeze-dried, spray freeze-dried, or in any other reconstitutable form. Various reconstitution media, cleansing or disinfective agents, or topical steriliants (alcohol wipes, iodine) can further be provided if desired.

The practitioner would then either fill the delivery device with the active agent and then deliver the agent by removing the removable release liner if present, and applying the device to the skin and deforming the deformable chamber with finger pressure to move the agent from the deformable chamber to a location between the porated disc and the skin; or, if the delivery device were pre-loaded, simply removing the removable release liner if present, applying the device to the skin and deform the deformable chamber with finger pressure to move the agent from the deformable chamber to a location between the porated disc and the skin.

Those skilled in the art will appreciate that the invention described herein is susceptible to variations and modifications other than those specifically described. The invention includes all such variation and modifications. The invention also includes all of the steps, features, formulations and compounds referred to or indicated in the specification, individually or collectively and any and all combinations or any two or more of the steps or features.

Each document, reference, patent application or patent cited in this text is not repeated in this text is merely for reasons of conciseness.

Any manufacturer's instructions, descriptions, product specifications, and product sheets for any products mentioned herein may be employed in the practice of the invention.

The invention described herein may include one or more range of values (eg. size, displacement and field strength etc). A range of values will be understood to include all values within the range, including the values defining the range, and values adjacent to the range which lead to the same or substantially the same outcome as the values immediately adjacent to that value which defines the boundary to the range.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs. The term "active agent" may mean one active agent, or may encompass two or more active agents.

Throughout this specification, unless the context requires otherwise, the word "comprise" or variations such as "comprises" or "comprising", will be understood to imply the inclusion of a stated integer or group of integers but not the exclusion of any other integer or group of integers.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs. The term "active agent" may mean one active agent, or may encompass two or more active agents.

### NON-LIMITING ILLUSTRATION OF THE INVENTION

Further features of the present invention are more fully described in the following description of several non-limiting embodiments thereof. This description is included solely for the purposes of exemplifying the present invention. It should not be understood as a restriction on the broad description of the invention as set out above. The description will be made with reference to the accompanying drawings in which:

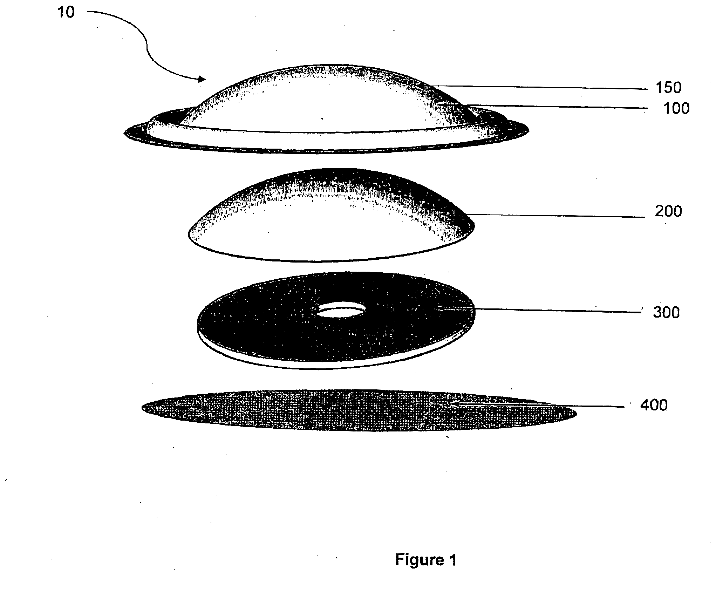

a) Figure 1 is an exploded view of a delivery device according to a first embodiment of the invention, showing the deformable chamber comprising one dome-shaped member, porated disc and removable release liner, and the active agent located within the reservoir formed by the deformable chamber;

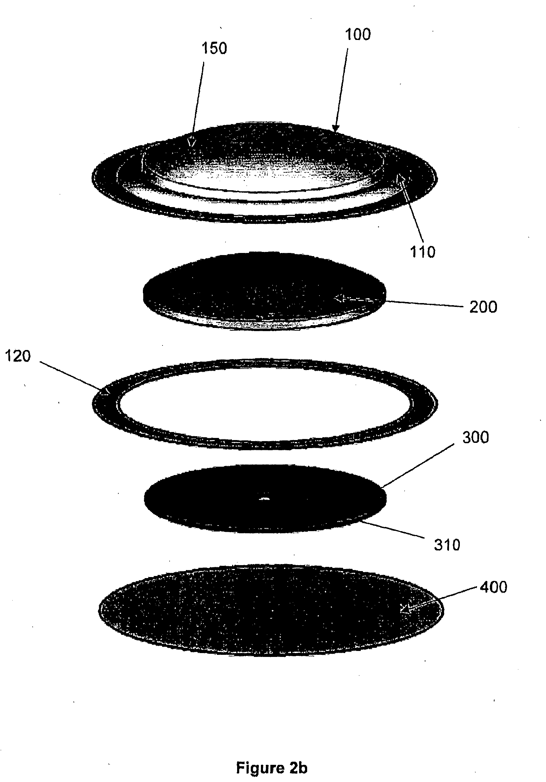

b) Figure 2a is a cut-away view and Figure 2b an exploded view of a delivery device according to a first embodiment, further showing the pore(s) in the porated disc, the transparent window ring and the adhesive ridge;

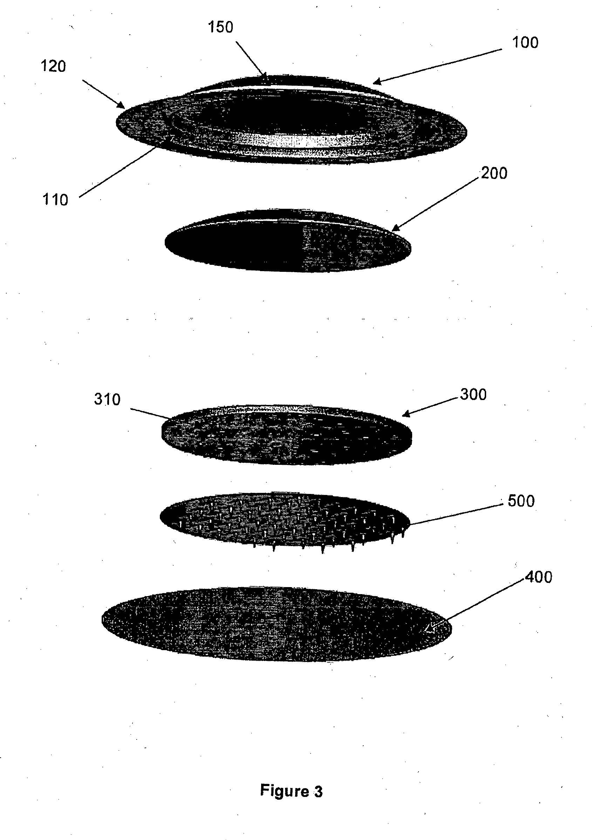

c) Figure 3 is an exploded view of a of a delivery device according to a second embodiment, showing the deformable chamber comprising one dome-shaped member, adhesive ridge, active agent located within the reservoir formed by the deformable chamber, porated disc, pore(s) in the porated disc, microneedle layer, removable release liner, transparent window ring;

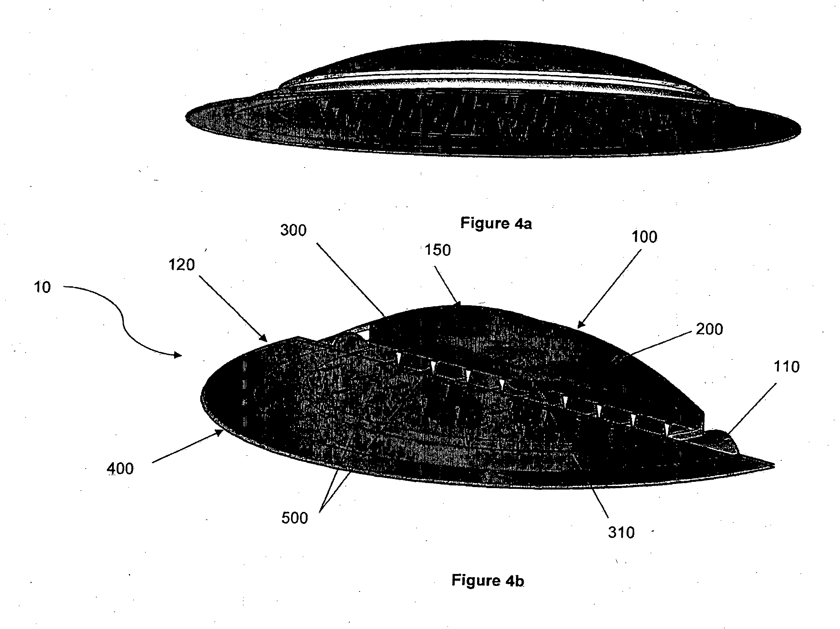

d) Figure 4a is a perspective view of a delivery device according to a second embodiment, showing the microneedles below the porated disc and a single pore in the middle of the porated disc; Figure 4b is a cut-away view of the same device;

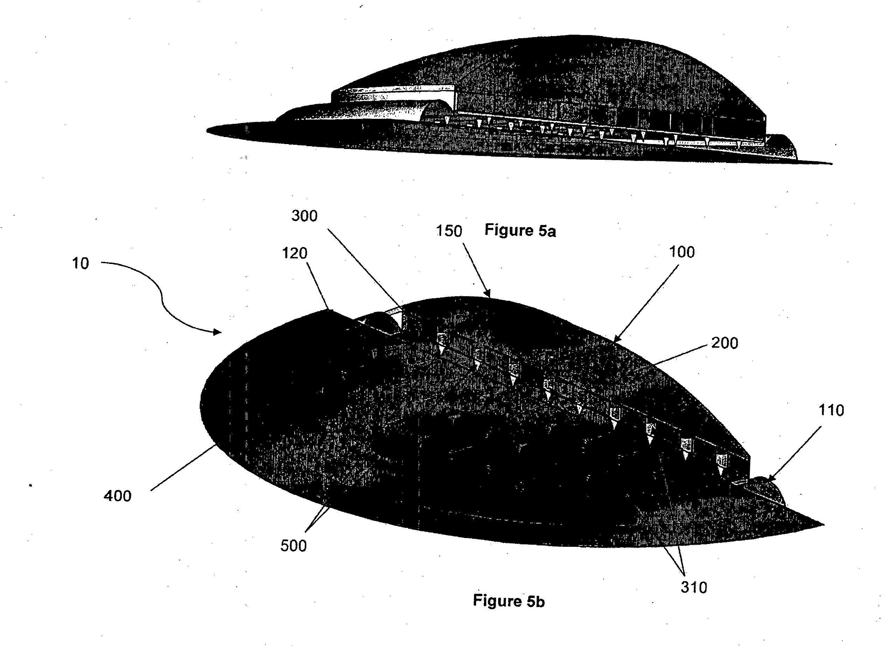

e) Figure 5a and 5b are cut-away views of a delivery device according to a second embodiment, showing the microneedles below the porated disc and multiple pores in the porated disc;

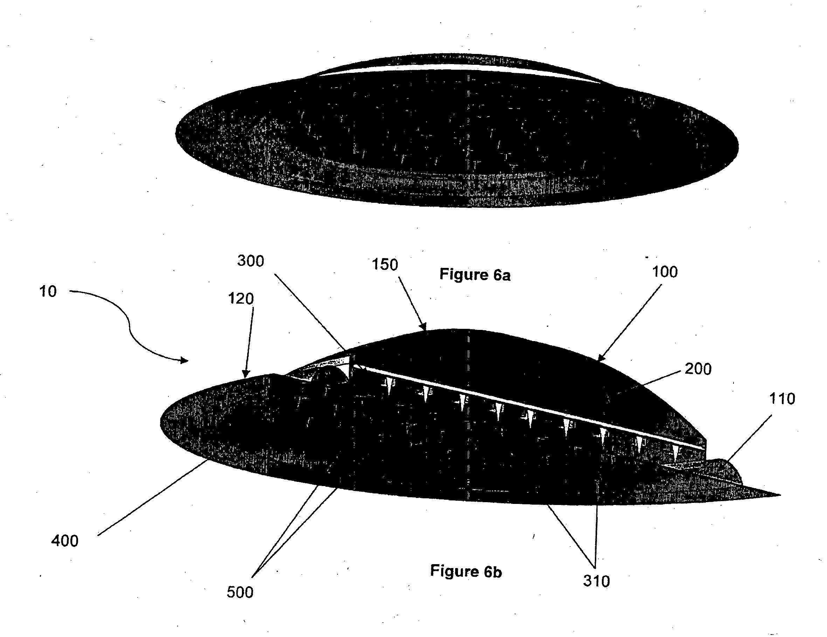

f) Figure 6a is a perspective view of a delivery device according to a second embodiment, showing the microneedles above the porated disc, penetrating through the multiple pores in the disc; Figure 6b is a cut-away view of the same device;

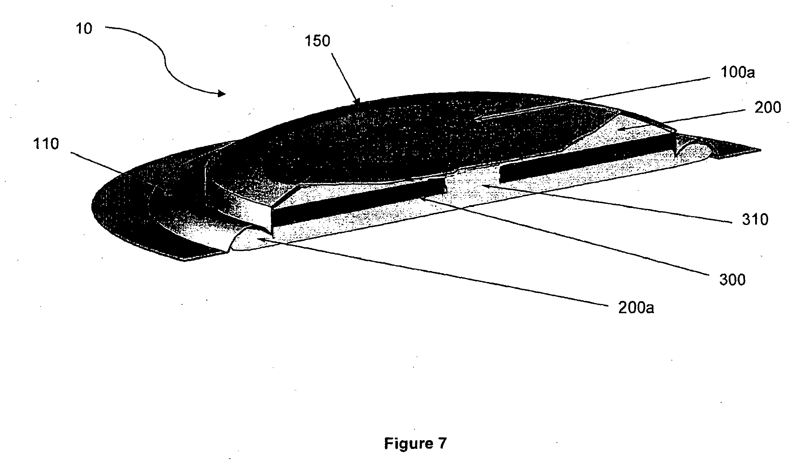

g) Figure 7 is a perspective view of a delivery device according to a first embodiment, wherein the deformable chamber comprising one dome-shaped member has been deformed by application of finger pressure and the active agent has moved from the reservoir formed by the deformable chamber through the pore(s) to the region between the porated disc and the skin and further into the transparent window ring;

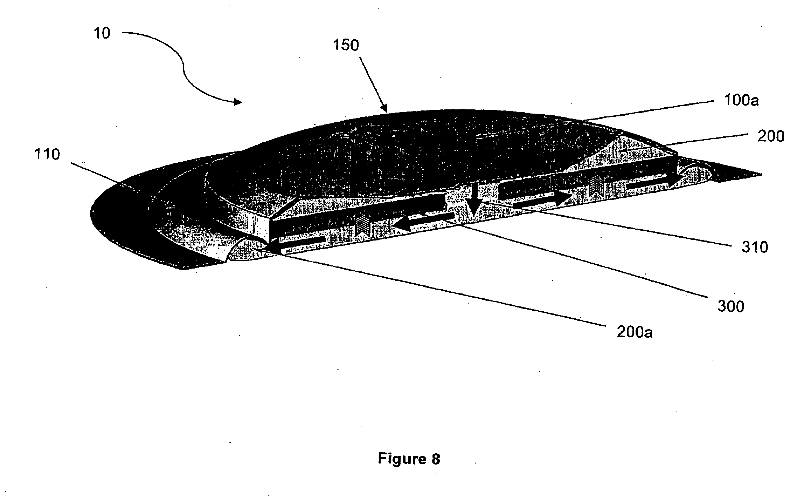

h) Figure 8 is a perspective view of a delivery device according to a first embodiment, with arrows showing the movement of the active agent from the reservoir formed by the deformable chamber through the pore(s) to the region between the porated disc and the skin in response to deformation of the chamber (black arrows), and movement of the porated disc up from the skin by the hydraulic action of the moving active agent (hatched arrows);

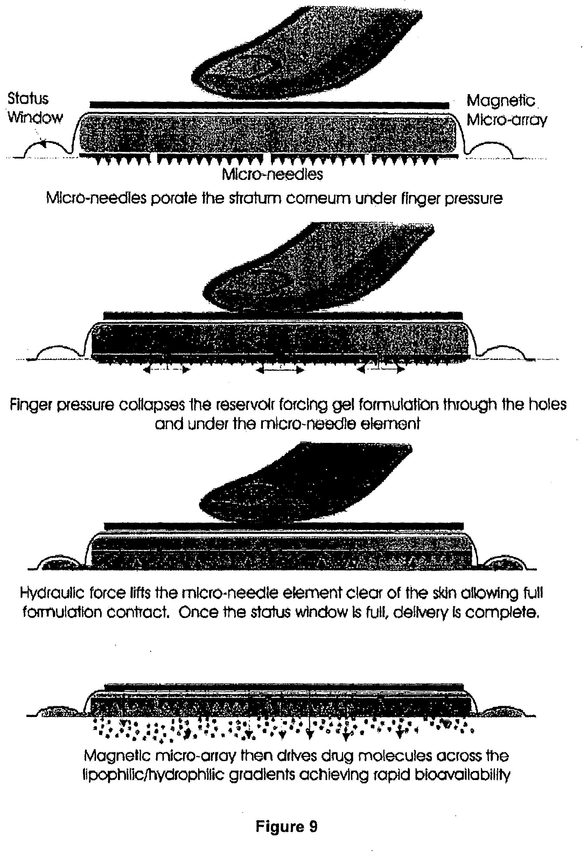

i) Figure 9 is a diagrammatic depiction of a delivery device according to a second embodiment in action;

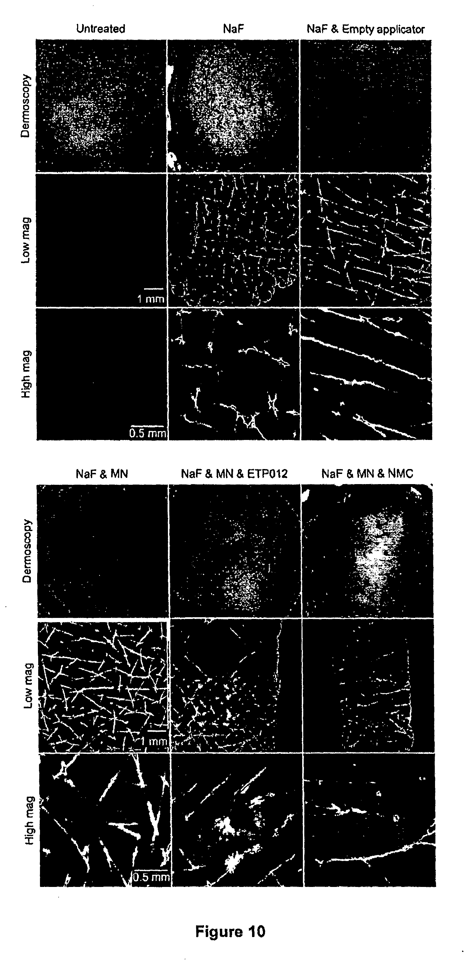

j) Figure 10 is a dermoscopy and confocal image of skin treated with a delivery device according to a second embodiment. Skin was treated with saline, sodium fluorescein (NaF), microneedles (MN), ETP012 plus MN or a non-magnetic control material (NMC). Dermoscopy images are shown in colour and LSCM images are shown at low and high magnification;

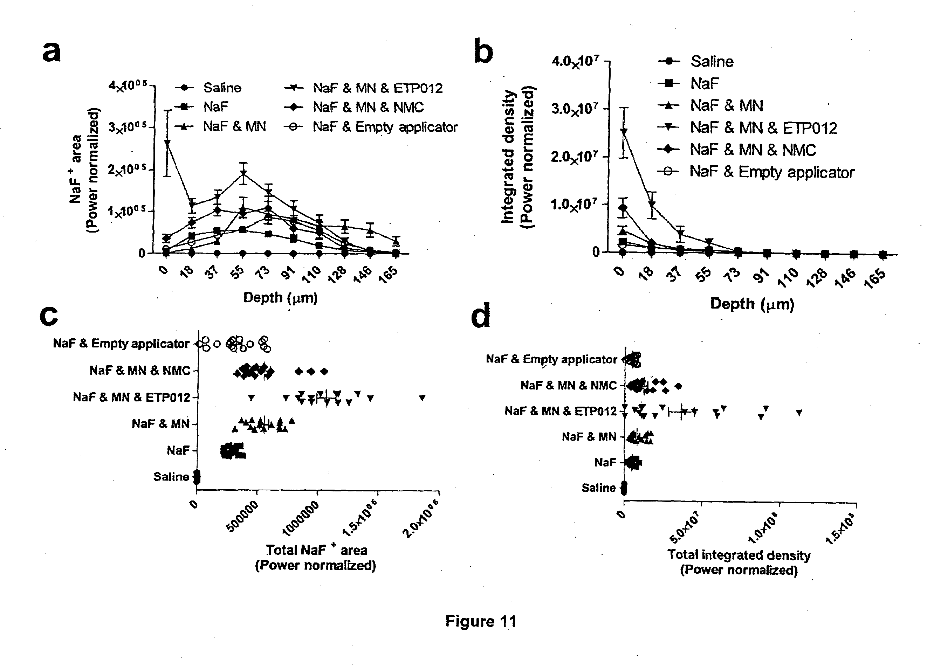

k) Figure 11 is a series of graphs of image analysis results from LSCM. Panel (a) shows the normalized size of the NaF positive (NaF+) areas at each depth. Panel (b) is a summary of that data wherein the values from Panel (a) were summed through the entire z-stack. Panel (c) represents the integrated density of the NaF+ areas at each depth analysed. The sum of those data is shown in Panel (d);
l) Figure 12 is a cut-away view of a delivery device according to a third embodiment of the invention, showing the double dome configuration comprising two dome-shaped members forming a sphere shaped deformable chamber, wall sections in contact with the sides of the deformable chamber, and a porated disc on the side of the chamber that is in contact with the skin when is use wherein the porated disc forms the skin facing member;
m) Figures 13a and 13b are views of a delivery device according to a fourth embodiment of the invention, with 13a showing the device prior to deformation with the active agent within the deformable chamber and 13b showing the chamber after deformation after the active agent has been dispensed. The device comprises the double dome configuration comprising two dome-shaped members forming a sphere shaped deformable chamber, wall sections in contact with the sides of the deformable chamber, a porated disc and a separate skin facing member;
n) Figure 14 is an exploded view of a delivery device according to a fourth embodiment of the invention, showing a spherical deformable chamber formed of two dome-shaped members surrounded by a spherical wall section which extends laterally to form a flat surface between the porated disc and the skin when in use, and a porated disc;

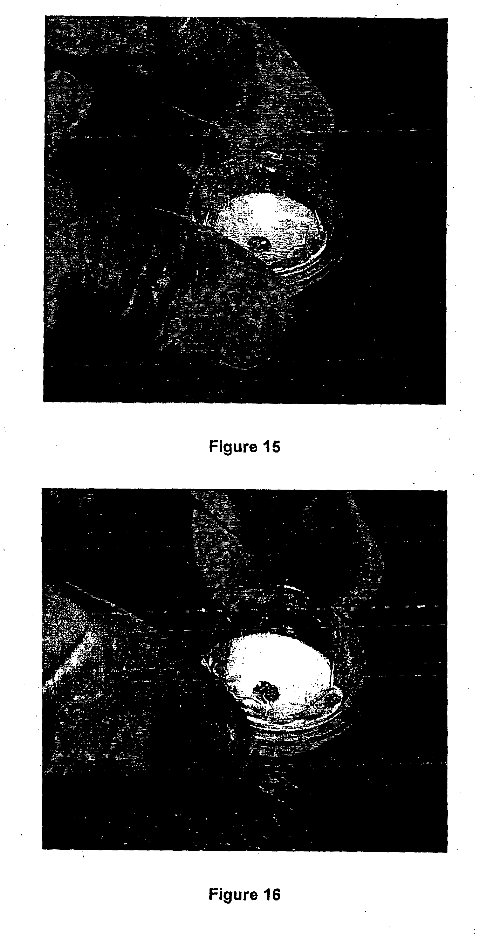

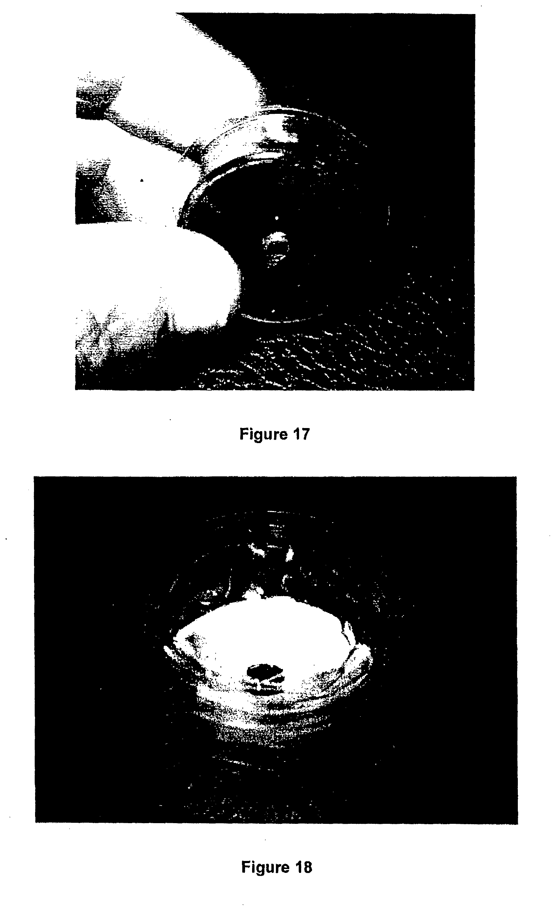

o) Figures 15 to 18 are photographs of a fourth embodiment of the invention, showing: Figure 15 - the device being held, Figure 16 - the device after deformation of the deformable dome, Figure 17 - the portion of the device that comes nto contact with the skin during use, showing the skin facing member through which is visible the porated disc, Figure 18 - the device with the porated disc.
p) Figures 19a and 19b are cut-away views of a delivery device according to a fifth embodiment of the invention, with 19a showing the dome-shaped member in a tensioned state (and the shape of the dome-shaped member in a relaxed state shown with a broken line) prior to deformation, with the active agent within the deformable chamber and 13b showing the chamber after deformation after the active agent has been dispensed with the dome-shaped member in a relaxed state (and the shape of the dome-shaped member in a tensioned state shown with a broken line).

Referring to Figures 1 to 4, there is provided a device for delivering an active agent to a dermal or mucosal surface 10 comprising deformable chamber 100 comprising one dome-shaped member 150 able to deform in a predicable manner to the application of finger pressure, a measured dose of active agent 200, a porated disc 300 having magnetophoretic properties and a removable liner 400. The active agent 200 is located between the deformable chamber 100 and the porated disc 300, whilst the porated disc 300 is located between the active agent 200 and the removable liner 400.

Referring to Figure 2a and 2b, there may be one or more than one pores 310 in the porated disc 300. The device 10 may further comprise an adhesive ridge 120 to provide adhesion to the skin and a transparent window ring 110 into which the active agent moves when the deformable chamber 100 is deformed and the active agent 200 moves through the pore(s) to the region between the porated disc 300 and the skin. The transparent window ring 110 serves as status window to show movement of active agent 200 from the deformable chamber 100 outwards under pressure.

The device 10 of Figure 1 and 2 is approximately 35mm in diameter, with the dome of the deformable chamber 100 being approximately 6mm high. The pore 310 is approximately 5mm in diameter. The deformable chamber 100 would hold a volume of active agent 200 of about 0.5ml.

As shown in Figures 3 to 6, the device may further optionally comprise a layer of micro-needles 500, which may be located either on the underside of porated disc 300 or above porated disc 300, penetrating through the pores in the disc.

In use, the user removes the removable release liner 400 and places the device on the skin. Finger pressure is applied to the top of the deformable chamber 100. In the case when the porated disc 300 has microneedles 500 on the underside, this finger pressure drives the micro-needles 500 into the stratum corneum, creating poration (Figure 9).

With reference to Figure 7, whether the delivery device has micro-needles or not, the finger pressure acts to deform deformable chamber 100 (shown in its deformed configuration 100a). As finger pressure increases, the dome shaped member 150 of deformable chamber 100 is deformed beyond its normal elastic limit until the crown or top of the chamber collapses downward to deformed configuration 100a. This action causes the active agent 200 to be expelled through pore(s) 310 in the porated disc 300, causing the active agent 200 to be re-distributed into the region between the skin and the porated disc 300 (active agent 200 is shown in its distributed configuration 200a). The displaced volume, caused by predicable deformation of the deformable chamber 100, is calculated to provide sufficient pressure to raise the porated disc 300 clear of the skin. The active agent 200 will fill this region between the skin and porated disc 300 and flow into the transparent window ring 110 to act as a clear visual indicator that the region between device 10 and the skin surface is now fully changed with distributed active agent 200a.

If the porated disc 300 comprised micro-needles 500 on its underside, then the initial pressure on the dome-shaped member 150 of deformable chamber 100 will serve to drive the micro-needles 500 into the skin and cause poration of the skin. Continued pressure on deformable chamber 100 and deformation of the chamber will cause active agent 200 to lift the porated disc 300 with micro-needles 500 clear of the skin and provide the necessary active agent to skin contact.

The action of pressure on, and deformation of, the dome-shaped member 150 of deformable chamber 100 will provide the optimum alignment of the skin poration zone and the device 10 active agent application zone, whilst maintaining sterility and excluding environmental contaminants that may otherwise induce unsafe or adverse immune responses. Furthermore, the porated disc 300, now superior to the active agent 200 (as active agent 200 is in contact with the skin) can act to enhance skin permeability and the transdermal delivery of the active agent.

Referring to Figure 8, the bold black arrows indicate the movement of active agent 200 from the deformed deformable chamber 100 (now in configuration 100a) through pore(s) 310 into the region between porated disc 300 and the skin surface, and into transparent window ring 110. The movement of active agent 200 has resulted in lifting of porated disc 300 from the skin surface as indicated by the textured arrows.

Referring to Figure 12, the deformable chamber 100 is shown in a spherical configuration, created by the juxtaposition of a first dome shaped member . 150 and a second dome shaped member 160. The device further comprises wall section 600 surrounding the deformable chamber 100 and being in contact with the largest longitudinal diameter of the deformable chamber such that the wall section 600 interconnects with the deformable chamber 100 via an interference fit or snap lock connection. The porated disc 300 is integral with the wall section 600.

Referring to Figure 13a the formable chamber 100 is shown in a spherical configuration, created by the juxtaposition of a first dome shaped member 150 and a second dome shaped member 160. The device further comprises wall section 600 surrounding the deformable chamber 100 and being in contact with the largest longitudinal diameter of the deformable chamber such that the wall section 600 interconnects with the deformable chamber 100. The skin facing member 610 is integral with the wall section 600 and the porated disc 300 is separate, being between the skin facing member 610 and the second dome-shaped member 160. In Figure 13b, the dome shaped member 150 has been deformed to configuration deformable chamber 100a, the collapsed dome, by pressure of fingertips on the top of the dome shaped member 150.

Referring to Figure 19a and 19b, finger pressure on the tensioned dome-shaped member 150 of the deformable chamber 100 of Figure 19a causes the dome-shaped member 150 to deform by rebounding to its relaxed state until the crown or top of the chamber collapses downward to deformed configuration 100a which is a relaxed state. This action causes the active agent 200 to be expelled through pore(s) in the porated disc 300, causing the active agent 200 to be re-distributed into the region between the skin and the porated disc 300 (in Figure 19b the active agent 200 is shown in its distributed configuration 200a). The tensioned deformable chamber 100 undergoes predicable deformation as it springs back to its relaxed state 100a.

### Example 1

### METHODS

Excised human skin handling: Human skin was collected from abdominoplasty surgery under Princess Alexandra Hospital Research Committee Approval No. 2007/197, administrated by the University of Queensland Human Ethics Committee. On receipt, the skin was wrapped in aluminium foil, inserted into a sealed bag with identification details, and stored at -20°C prior to use.

After removal from the freezer, the identification details of the skin were recorded and the skin left at room temperature for 20 minutes to partially thaw. A different skin piece was selected for each replicate. One inch circles of skin were cut using a punch and a press, and trimmed with a pair of curved surgery scissors. After excision, the skin samples were placed on top of gauze hydrated with 5mL of saline and the samples equilibrated for 30 minutes before gathering transepidermal water loss (TEWL) readings.

TEWL: TEWL measurements were performed using AquaFlux AF200 closed chamber evaporimeter (Biox Systems Ltd., London, UK). AquaFlux V6.2 software was used to analyse the data. After calibration, the TEWL probe was positioned in the centre of the skin sample and the handle maintained perpendicular to the surface of the skin. A slight pressure was applied to the skin through the probe and then the TEWL device activated. Data logging automatically ceased once the rate of change of the TEWL reading dropped below a specified amount or the test time had elapsed its specified duration. TEWL measurements were taken before and after microneedle treatment.

Microneedle Fabrication: The configuration and geometries of the microneedle arrays are 700 µm microneedle length × 250 µm width × 50µm thickness. There were 3 microneedles per 5mm plate. The plates were assembled in banks of 2 with 2mm spacing. A LaserPro S290 (M2 Lasers, Brisbane, QLD) laser milling machine was used to cut the 50µm thick 304 stainless steel sheet to the microneedle specifications. The cutting laser is a 20W source coupled to a cutting head with fibre optic cable with a resolution of 1000dpi. Quality control was done on the microneedles before application and the observed configuration was 703.1±16.1µm long and 257.8±9.4µm wide with a tip at a 55° that is 250µm long.

Microneedle Treatment: A piece of gauze was folded in half 4 times and then the skin sample placed on top. In-situ skin tension was approximately simulated by stretching the skin over the gauze and pinning in place. A pre-prepared microneedle impact applicator was then used to apply the microneedle to the skin at speed (approximately 3m·s-1). This applicator consisted of a housing, a springloaded plunger and the banks of laser cut microneedles (two 3-needle banks gave a total of 6 microneedles). The banks were mounted into the plunger which was then drawn back, loading the spring, and held in place using a pin. Upon removal of the pin, the plunger accelerated towards the skin, driving the microneedles through the stratum corneum and into the viable epidermis and dermis.

Dye incubation in Franz Cells: A Franz cell was prepared by applying vacuum grease to the sealing surfaces of the receptor and donor. A stirring magnet was then added to the receptor. The skin sample was placed on the receptor with the skin side facing upwards. The donor was assembled on top and fastened in place using a collar. The receptor was then filled with saline. During this step, steps were taken to ensure no air bubbles remained in the receptor. A volume of 300µL Sodium Fluorescein (NaF) at 1mg/mL in saline was pipetted onto the centre of the skin in the Franz cell.

Magnetophoresis: A rubber o-ring (o.d. 10mm and i.d. 7mm) was placed onto the skin surface in the Sodium Fluorescein solution and the magnet or non-magnetic control (NMC) was then laid on top. The o-ring served to maintain the dye reservoir on the surface of the skin and prevent the magnet from occluding the pores. After dye/magnet treatment, vacuum grease was added to the top of the donor cell and a glass cover slip placed on this surface to seal the unit, thus minimizing evaporation. The Franz cell was then placed into a circulating, heated water bath at a temperature of between 34°C and 35°C and covered in aluminium foil. The Franz cells were incubated in this bath for 15 minutes. The Franz cells were then disassembled and the skin samples placed back in the hydrating weight boat and covered with aluminium foil to prevent photobleaching.

Laser scanning confocal microscopy (LSCM): To prepare the samples for LSCM, a cut was made around the imprint made by the Franz cell. This reduced the size of the skin sample to allow easy and reproducible mounting into the LSCM stage. A small drop of immersion oil was added to the skin surface of the excised skin samples and massaged. A small compressive load was applied to the skin sample in the form of a custom made sample cup to ensure a flat skin surface for imaging. A dermoscopic image was first taken. This gave a macroscopic image of the skin surface and aided in accurate targeting of the microneedle pores during LSCM imaging. The sample was then mounted into the LSCM and, using reflectance confocal microscopy (RCM), the skin surface was located. After adjusting the location so that a plane just above the skin surface was visible, the zero depth was set. The LSCM machine was then switched to the fluorescent mode with 488nm excitation and 550±88nm emission band was recorded. A mosaic/z-stack was obtained consisting of ten 7mm X 7mm layers with a 20µm step between each layer and each image was 0.5x0.5mm2 at 1000x1000px2. After completion, the LSCM machine was switched back to RCM mode and an identical stack acquired.

Image analysis: The image data names were encoded to blind the image analysis researcher. Once the analysis was complete, the codes were revealed and the data organized into groups. Each mosaic image was reduced in size to 10% and saved as a jpg file using Adobe Photoshop. Image J (NIH, Bethesda, MD) was then used to quantify fluorescence intensity and dye positive areas. Once a z-stack of mosaics were loaded into Image J, a threshold was applied at 100-255 to a 500x500px (2.5x2.5mm2) area of interest. The "Analyse Particles" function was applied with a size range restriction of 500-infinity to exclude furrows and background. The output was "Positive Area" and "Integrated Intensity". These values were normalized to laser power by dividing the output with the laser power.

### RESULTS

TEWL: Before any treatment the average TEWL was 11.3±3.7 g/m2h. After microneedle treatment the TEWL increased to 17.9±3.7 g/m2h and this was a significant (p=0.0002) increase as determined by an Unpaired t-test with Welch's correction applied to these two groups. However, there were no significant differences between treatment groups analysed with a 1 way ANOVA (Kruskal-Wallis test) with a Dunns post-test. This is likely due to the small sample size of n=3 per group. Table 1 shows the mean TEWL values before and after microneedle treatment of each group, in addition to the average TEWL change. The changes in TEWL between the ETP012 and other microneedle groups were not significant, suggesting all microneedling was consistent in terms of TEWL.

**Table 1 TEWL values before and after microneedle treatment. The treatment groups include treatment with fluorescein (NaF), microneedles (MN), ETP012 and the non-magnetic control (NMC).**

| | **Before** | **After** | **Delta** |
|---|---|---|---|
| **Group** | *Ave* ± *StDev* | *Ave* ± *StDev* | *Ave* ± *StDev* |
| Saline | 15.4 ± 5.2 | 16.6 ± 8.3 | 1.2 ± 32 |
| NaF | 11.8 ± 0.9 | 11.0 ± 1.4 | -0.8 ± 0.7 |
| NaF & MN | 9.6 ± 0.8 | 16.2 ± 3.4 | 6.6 ± 3.3 |
| NaF & MN & ETP012 | 12.2 ± 1.4 | 20.2 ± 1.7 | 8.0 ± 1.7 |
| NaF & MN & NMC | 9.7 ± 1.1 | 17.4 ± 3.0 | 7.7 ± 4.0 |
| NaF & NMC | 9.3 ± 0.7 | 9.6 ± 0.5 | 0.3 ± 0.3 |

Confocal Imaging: Dermoscopy imaging clearly showed microneedle pores in microneedle treated samples (MN, Figure 10). RCM also confirmed the presence of microneedle pores (data not shown). LSCM revealed a NaF fluorescence pattern that consisted of primarily furrow localization in all NaF treated groups. One group was treated with the microneedle applicator without microneedles to control for any barrier disruption from the high velocity impact. These data are marked "NaF & Empty applicator" and show a fluorescence pattern similar to NaF alone where there was no penetration of the dye beyond the stratum corneum. The only cases where there dye penetration occurred were those samples treated with microneedles (Figure 10, bottom panel). In all samples treated with microneedles, the dye diffused through the stratum corneum and epidermis, into the dermis. All of the fluorescence images shown in Figure 10 are from 73µm deep, which corresponds to the superficial dermis. Visually, the group with NaF & MN & EPT012 showed the highest levels of radial diffusion and depth of NaF penetration. However, the overall levels of fluorescence were quite variable from one microneedle pore to the next.

Image Analysis: Image analysis was done to identify the normalized area positive for NaF fluorescence. A threshold served to reduce the confounding influence of NaF positive furrow data and select highly fluorescent areas. The resulting data shown in Figure 11a and c illustrate that there was a clear increase in NaF positive area in skin treated with the combination of microneedles and ETP012. In Figure 11a, the NaF positive area for skin treated with microneedles and ETP012 remained above background levels (i.e. microneedles with the non-magnetic control (NMC)) to a depth of ∼100µm. This suggests that the ETP012 effects were limited to the stratum corneum, epidermis and uppermost dermis.

Figure 11c shows a summary of the NaF positive area data that sums the data from the entire z-stack. This comparison clearly indicates a positive effect of ETP012 over the non-magnetic control material.

Integrated density is a measure of the intensity of the NaF signal and the data shown in Figure 11b clearly indicates that ETP012 treated skin had higher levels of fluorescence in the stratum corneum and epidermis than all other groups. These data suggest that there was enhanced influx of NaF in the top 37µm of skin. Figure 11d summarizes the results from all groups and again shows that the ETP012 treatment clearly affected NaF fluorescence localization, but also showed a corresponding increase in variability.

We observed no differences between the treatment groups with dermoscopy or RCM with the exception of the pores in those groups treated with microneedles. LSCM with ETP012 had a positive effect on dye penetration and diffusion, while also increasing the inter- and intra-sample variability. This resulted in clearly different dye penetration kinetics for the ETP012 group versus all other controls, showing that ETP012 treatment improved microneedle enhanced drug delivery and variability.

## Claims

1. A device (10) for delivery of a measured or predetermined dose of an active agent (200) to a dermal or mucosal surface comprising:
a) a deformable chamber (100) comprising at least one dome-shaped member (150) defining a reservoir for the active agent; and
b) a porated disc (300) wherein the deformable chamber is a spherical or semi-circular shape in vertical section in relation to the longitudinal plane of the porated disc when in use against the skin and wherein the volume of the deformable chamber is predicably changed by the application of external pressure to the deformable dome-shaped member such that the dome-shaped member of the chamber distal from the skin when in use deforms to adopt the shape of either (a) the porated disc if the chamber is semi-circular in shape in vertical section or (b) the shape of the second dome-shaped member adjacent to the skin if the chamber is spherical in shape in vertical section, **characterized in that** the porated disc has magnetophoretic properties.

2. A device according to claim 1, wherein the device has one dome-shaped member (150) and wherein the deformable chamber is a semi-circular shape in vertical section in relation to the longitudinal plane of the porated disc when in use against the skin.

3. A device according to claim 1, wherein the device has two dome-shaped members (150, 160) and wherein the deformable chamber is substantially spherical shape in vertical section in relation to the longitudinal plane of the porated disc when in use against the skin.

4. A device according to any one of claims 1 to 3 wherein the porated disc incorporates microneedles (500).

5. A method for delivery of a measured or predetermined dose of a cosmetic agent to a dermal or mucosal surface comprising the step of:
a) applying a device (10) for delivering a cosmetic agent to a dermal or mucosal surface to the dermal or mucosal surface; and
b) applying pressure to the device to cause deformation of a deformable chamber (100)
wherein the device comprises: a deformable chamber comprising at least one dome-shaped member (150) defining a reservoir for the cosmetic agent (200) and a porated disc (300) wherein the deformable chamber is a spherical or semi-circular shape in vertical section in relation to the longitudinal plane of the porated disc when in use against the skin and wherein the volume of the deformable chamber is predicably changed by the application of external pressure to the deformable dome-shaped member such that the dome-shaped member of the chamber distal from the skin when in use deforms to adopt the shape of either (a) the porated disc if the chamber is semi-circular in shape in vertical section or (b) the shape of the second dome-shaped member adjacent to the skin if the chamber is spherical in shape in vertical section, **characterized by** the porated disc having magnetophoretic properties.

6. A method according to claim 5 wherein the device has one dome-shaped member and wherein the deformable chamber is a semi-circular shape in vertical section in relation to the longitudinal plane of the porated disc when in use against the dermal or mucosal surface.

7. A method according to claim 5 wherein the device has two dome-shaped members and wherein the deformable chamber is substantially spherical shape in vertical section in relation to the longitudinal plane of the porated disc when in use against the dermal or mucosal surface.

8. A method according to any one of claims 5 to 7 wherein the porated disc incorporates microneedles (500).

9. A device according to claim 1 wherein the magnetophoretic porated disc comprises a magnetic array comprising one or more pairs of displaced dipolar magnetic elements.

10. A device according to claim 1 wherein the magnetophoretic porated disc comprises a magnetic array comprising one or more pairs of displaced dipolar magnetic elements encapsulated in a flexible matrix.

11. A device according to claim 1 further comprising a removable release liner (400).

12. A device according to claim 1 further comprising an adhesive ring (20) wherein during use the adhesive ring removably adheres the device to the dermal or mucosal surface.

13. A device according to claim 1 wherein the dome-shaped member of the deformable chamber is in a tensioned state prior to deformation by application of external pressure.

14. A device according to claim 1 further comprising an absorbent delivery surface, located between the porated disc and the dermal or mucosal surface when the device is in use.

15. A kit comprising a device according to claim 1.

## Patentansprüche

1. Vorrichtung (10) zur Verabreichung einer abgemessenen oder vorbestimmten Dosis eines Wirkstoffs (200) an eine Haut- oder Schleimhautoberfläche, umfassend:
a) eine deformierbare Kammer (100), die mindestens ein kuppelförmiges Glied (150) umfasst, das ein Reservoir für den Wirkstoff definiert, und
b) ein poriges Plättchen (300),
wobei die deformierbare Kammer im vertikalen Schnitt in Bezug auf die Längsebene des porigen Plättchens kugel- oder halbkreisförmig ist, wenn sie sich an der Haut im Einsatz befindet, und wobei das Volumen der deformierbaren Kammer durch die Ausübung von externem Druck auf das deformierbare kuppelförmige Glied vorhersehbar geändert wird, so dass sich das kuppelförmige Glied der Kammer im Einsatz distal von der Haut deformiert, um entweder (a) die Form des porigen Plättchens anzunehmen, wenn die Kammer im vertikalen Schnitt halbkreisförmig ist, oder (b) die Form des der Haut benachbarten zweiten kuppelförmigen Glieds anzunehmen, wenn die Kammer im vertikalen Schnitt kugelförmig ist, **dadurch gekennzeichnet, dass** das porige Plättchen magnetophoretische Eigenschaften hat.

2. Vorrichtung nach Anspruch 1, wobei die Vorrichtung ein kuppelförmiges Glied (150) hat und wobei die deformierbare Kammer im vertikalen Schnitt in Bezug auf die Längsebene des porigen Plättchens, wenn sie sich an der Haut im Einsatz befindet, halbkreisförmig ist.

3. Vorrichtung nach Anspruch 1, wobei die Vorrichtung zwei kuppelförmige Glieder (150, 160) hat und wobei die deformierbare Kammer im vertikalen Schnitt in Bezug auf die Längsebene des porigen Plättchens, wenn sie sich an der Haut im Einsatz befindet, im Wesentlichen kugelförmig ist.

4. Vorrichtung nach einem der Ansprüche 1 bis 3, wobei in das porige Plättchen Mikronadeln (500) eingegliedert sind.

5. Verfahren zur Verabreichung einer abgemessenen oder vorbestimmten Dosis eines Kosmetikstoffs an eine Haut- oder Schleimhautoberfläche, umfassend den Schritt des
a) Aufbringens einer Vorrichtung (10) zur Verabreichung eines Kosmetikstoffs an eine Haut- oder Schleimhautoberfläche an die Haut- oder Schleimhautoberfläche und
b) Ausübens von Druck auf die Vorrichtung, um eine Deformierung einer deformierbaren Kammer (100) zu veranlassen,
wobei die Vorrichtung Folgendes umfasst:
eine deformierbare Kammer, die mindestens ein kuppelförmiges Glied (150) umfasst, das ein Reservoir für den Kosmetikstoff (200) definiert, und ein poriges Plättchen (300), wobei die deformierbare Kammer im vertikalen Schnitt in Bezug auf die Längsebene des porigen Plättchens kugel- oder halbkreisförmig ist, wenn sie sich an der Haut im Einsatz befindet, und wobei das Volumen der deformierbaren Kammer durch die Ausübung von externem Druck auf das deformierbare kuppelförmige Glied vorhersehbar geändert wird, so dass sich das kuppelförmige Glied der Kammer im Einsatz distal von der Haut deformiert, um entweder (a) die Form des porigen Plättchens anzunehmen, wenn die Kammer im vertikalen Schnitt halbkreisförmig ist, oder (b) die Form des der Haut benachbarten zweiten kuppelförmigen Glieds anzunehmen, wenn die Kammer im vertikalen Schnitt kugelförmig ist, **dadurch gekennzeichnet, dass** das porige Plättchen magnetophoretische Eigenschaften hat.

6. Verfahren nach Anspruch 5, wobei die Vorrichtung ein kuppelförmiges Glied hat und wobei die deformierbare Kammer im vertikalen Schnitt in Bezug auf die Längsebene des porigen Plättchens, wenn sie sich an der Haut- oder Schleimhautoberfläche im Einsatz befindet, halbkreisförmig ist.

7. Verfahren nach Anspruch 5, wobei die Vorrichtung zwei kuppelförmige Glieder hat und wobei die deformierbare Kammer im vertikalen Schnitt in Bezug auf die Längsebene des porigen Plättchens, wenn sie sich an der Haut- oder Schleimhautoberfläche im Einsatz befindet, im Wesentlichen kugelförmig ist.

8. Verfahren nach einem der Ansprüche 5 bis 7, wobei in das porige Plättchen Mikronadeln (500) eingegliedert sind.

9. Vorrichtung nach Anspruch 1, wobei das magnetophoretische porige Plättchen eine magnetische Anordnung umfasst, die ein oder mehrere Paare verschobener dipolarer magnetischer Elemente umfasst.

10. Vorrichtung nach Anspruch 1, wobei das magnetophoretische porige Plättchen eine magnetische Anordnung umfasst, die ein oder mehrere Paare verschobener dipolarer magnetischer Elemente umfasst, die in einer flexiblen Matrix eingekapselt sind.

11. Vorrichtung nach Anspruch 1, ferner umfassend eine entfernbare Abziehschutzfolie (400).

12. Vorrichtung nach Anspruch 1, ferner umfassend einen Klebering (20), wobei der Klebering die Vorrichtung während der Verwendung entfernbar an die Haut- oder Schleimhautoberfläche klebt.

13. Vorrichtung nach Anspruch 1, wobei das kuppelförmige Glied der deformierbaren Kammer vor der Deformierung durch Ausübung von externem Druck in einem gespannten Zustand ist.

14. Vorrichtung nach Anspruch 1, ferner umfassend eine saugfähige Verabreichungsoberfläche, die zwischen dem porigen Plättchen und der Haut- oder Schleimhautoberfläche angeordnet ist, wenn die Vorrichtung im Einsatz ist.

15. Kit, umfassend eine Vorrichtung nach Anspruch 1.

## Revendications

1. Dispositif (10) pour administrer une dose mesurée ou prédéterminée d'un agent actif (200) à une surface dermique ou muqueuse comprenant :
a) une chambre déformable (100) comprenant au moins un élément (150) en forme de dôme délimitant un réservoir pour l'agent actif à l'air ; et
b) un disque poreux (300),
dans lequel la chambre déformable est de forme sphérique ou semi-circulaire en coupe verticale par rapport au plan longitudinal du disque poreux quand elle est utilisée contre la peau ; et
dans lequel le volume de la chambre déformable est de manière prévisible modifié par l'application d'une pression externe à l'élément déformable en forme de dôme de telle sorte que l'élément en forme de dôme de la chambre distal de la peau quand il est utilisé se déforme pour adopter la forme de soit (a) le disque poreux si la chambre est de forme semi-circulaire en coupe verticale soit (b) la forme du second élément en forme de dôme adjacent à la peau si la chambre est de forme sphérique en coupe verticale,
**caractérisé en ce que** le disque poreux présente des propriétés magnétophorétiques.

2. Dispositif selon la revendication 1, dans lequel le dispositif comporte un élément (150) en forme de dôme et
dans lequel la chambre déformable est de forme semi-circulaire en coupe verticale par rapport au plan longitudinal du disque poreux quand elle est utilisée contre la peau.

3. Dispositif selon la revendication 1, dans lequel le dispositif comporte deux éléments (150, 160) en forme de dôme et
dans lequel la chambre déformable est de forme sensiblement sphérique en coupe verticale par rapport au plan longitudinal du disque poreux quand elle est utilisée contre la peau.

4. Dispositif selon l'une quelconque des revendications 1 à 3, dans lequel le disque poreux incorpore des micro-aiguilles (500).

5. Procédé pour administrer une dose mesurée ou prédéterminée d'un agent cosmétique à une surface dermique ou muqueuse, comprenant les étapes consistant à :
a) appliquer un dispositif (10) pour administrer un agent cosmétique à une surface dermique ou muqueuse à la surface dermique ou muqueuse ; et
b) appliquer une pression au dispositif pour provoquer la déformation d'une chambre déformable (100), dans lequel le dispositif comprend une chambre déformable comprenant au moins un élément (150) en forme de dôme délimitant un réservoir pour l'agent cosmétique (200) et un disque poreux (300) ;
dans lequel la chambre déformable est de forme sphérique ou semi-circulaire en coupe verticale par rapport au plan longitudinal du disque poreux quand elle est utilisée contre la peau ; et
dans lequel le volume de la chambre déformable est de manière prévisible modifié par l'application d'une pression externe à l'élément déformable en forme de dôme de telle sorte que l'élément en forme de dôme de la chambre distal de la peau quand il est utilisé se déforme pour adopter la forme de soit (a) le disque poreux si la chambre est de forme semi-circulaire en coupe verticale soit (b) la forme du second élément en forme de dôme adjacent à la peau si la chambre est de forme sphérique en coupe verticale,
**caractérisé en ce que** le disque poreux présente des propriétés magnétophorétiques.

6. Procédé selon la revendication 5, dans lequel le dispositif comporte un élément en forme de dôme et
dans lequel la chambre déformable est de forme semi-circulaire en coupe verticale par rapport au plan longitudinal du disque poreux quand elle est utilisée contre la surface dermique ou muqueuse.

7. Procédé selon la revendication 5, dans lequel le dispositif comporte deux éléments en forme de dôme et dans lequel la chambre déformable est de forme sensiblement sphérique en coupe verticale par rapport au plan longitudinal du disque poreux quand elle est utilisée contre la surface dermique ou muqueuse.

8. Procédé selon l'une quelconque des revendications 5 à 7, dans lequel le disque poreux incorpore des micro-aiguilles (500).

9. Dispositif selon la revendication 1, dans lequel le disque poreux magnétophorétique comprend un réseau magnétique comprenant une ou plusieurs paires d'éléments magnétiques dipolaires déplacés.

10. Dispositif selon la revendication 1, dans lequel le disque poreux magnétophorétique comprend un réseau magnétique comprenant une ou plusieurs paires d'éléments magnétiques dipolaires déplacés encapsulés dans une matrice flexible.

11. Dispositif selon la revendication 1, comprenant en outre une doublure amovible (400) de libération.

12. Dispositif selon la revendication 1, comprenant en outre un anneau adhésif (20),
dans lequel, pendant l'utilisation, l'anneau adhésif fait coller de façon amovible le dispositif à la surface dermique ou muqueuse.

13. Dispositif selon la revendication 1, dans lequel l'élément en forme de dôme de la chambre déformable est en tension avant sa déformation par application d'une pression externe.

14. Dispositif selon la revendication 1, comprenant en outre une surface absorbante d'administration, située entre le disque poreux et la surface dermique ou muqueuse quand le dispositif est utilisé.

15. Nécessaire comprenant un dispositif selon la revendication 1.
